# EUROPEAN PATENT APPLICATION

(11) **EP 2 508 661 A2**
(43) Date of publication of application: **10.10.2012**
(21) Application number: 11792611.3
(22) Date of filing: 15.04.2011
(51) Int. Cl.: D04C 1/02, D04C 1/06, A61L 17/10, A61L 17/14

(54) **METHOD FOR MANUFACTURING A SURGICAL SUTURE**

(30) Priority: 09.06.2010 KR 20100054585
(71) Applicant: Meta Biomed Co., Ltd., Oksan-myeon, Cheongwon-gun Chungbuk 363-911 (KR)
(72) Inventor: YOO, Yeon Chun, Cheongju-si Chungbuk 360-190 (KR); PARK, Heung Su, Cheongju-si Chungbuk 361-850 (KR); KIM, Joon Hyung, Cheongwon-gun Chungbuk 363-911 (KR); CHON, Joon Sub, Jeonju-si Jeonbuk 561-856 (KR); LEE, Do Kyoung, Cheongju-si Chungbuk 360-210 (KR); CHOI, Jae Woon, Cheongju-si Chungbuk 361-865 (KR)
(74) Representative: Hocking, Adrian Niall
(86) International application number: PCT/KR2011/002691
(87) International publication number: WO 2011/155700

(57) **Abstract**

The present disclosure relates to a method of manufacturing a biodegradable surgical suture with a distal end shaped like a loop by using a twist braider. The surgical suture enables a doctor to easily tie the suture with a knot in a bodily region through which a needle passes after the needle passes through a bodily incision to be sutured once without making the knot.

## Description

### Technical Field

The present invention relates to a surgical suture with a distal end shaped like a loop.

### Background Art

An existing surgical suture is manufactured by simply connecting yarn to a needle. Through the improvement of such a simple method of manufacturing the existing surgical suture, the present invention for a suture with a distal end shaped like a loop has been conceived, so that the distal end of the surgical suture is easily knotted when an incision is sutured with the surgical suture (especially, in surgery using an endoscope).

Hereinafter, a background art will be described in detail.

A surgical suture is classified into an absorbable suture and a non-absorbable suture. A non-absorbable suture is mainly used for cardiac surgery, vascular surgery, and orthopedic surgery. In the meantime, a biodegradable suture is mainly used for plastic surgery, fixing of skin, and suturing of soft tissue. For example, a suture made of polyethylene, terephthalate, nylon, polypropylene, or silk is a non-absorbable suture, and a well-known suture made of 1,4-dioxane-2-one, epsilon-caprolactone, glycolide, L(-)lactide, or D(+)lactide is an absorbable suture.

A surgical suture is generally manufactured by attaching a needle to a suture according to a usage. However, there is a special case of a sale of a surgical suture attached to no needle or a surgical suture including needles attached to both sides thereof.

A suture is mostly used for suturing tissue in a surgical operation or fixing an inserted implant to tissue. Further, a surgical suture is manufactured in various sizes for various surgical operations, and a size of the suture used is mainly determined according to a strength and state of the tissue. A size of a surgical suture is manufactured in a size ranging from USP (United States Pharmacopeia) 3 and 4 to 7-0 (a standard in the Food and Drug Association in USA).

A surgical suture is manufactured by a braiding method and a monofilament method depending on a manufacturing method and a process of manufacturing a surgical suture generally includes three steps.

In the first step, a biodegradable suture is manufactured by the monofilament or braiding method. In the second step, the manufactured suture is attached to a needle. In the third step, the needle-attached suture is Ethylene Oxide (E.O) sterilized or gamma sterilized and put into a carton box. According to an existing method of manufacturing multifilament suturing yarn in the first step, the surgical suture combined with the needle is manufactured by braiding Full Draw Yarn (FDY) manufactured by a spinning apparatus with a braider, cutting the manufactured suturing yarn in a size of 750 mm, attaching both distal ends with a bioadhesive for prevention of the suture from being untightened, and inserting one side of the yarn in an eye of the needle and compressing the needle. The manufactured needle-combined surgical suture is E.O sterilized, to be packed and sold.

Hereinafter, a suture with a distal end shaped like a loop directly related to the present invention will be described. In suturing an incision by using the needle-combined surgical suture (especially, in surgery using an endoscope), the suture is knotted after passing tissue once for preventing the suture from being untightened. The suture is knotted using an endoscope, so that a high-level technical training is required and it is very difficult to knot the suture.

An endoscope was first used when an Arab doctor, Abulkasim, inserted a reflector reflecting light into a vagina, and had been gradually used for a nasal sinuses examination and a urinary bladder examination. The endoscope for an examination could be used for a region having a cavity in a body. However, a cystoscope was first used in 1910 by a Swedish doctor, Jacobaeus, for an examination of the abdominal cavity.

Endoscopic surgery had been developed from a diagnostic purpose to a surgical purpose by the beginning of the 1980s from the late 1970s, and a German surgeon, Kurt Semm, served a role as a pioneer in laparoscopic surgery. By the late 1980s, laparoscopic surgery was revolutionarily developed according to the development of medical technology, so that a new paradigm of a minimally invasive surgery developed from a laparotomy was created.

Endoscopic surgery is progressed by inserting a device equipped with an endoscope including a light and an imaging device into a body through a cavity after incising a small region and diagnosing a region for surgery and performing the surgery using tongs. A very small region is incised according to the development of the endoscopic surgery method, so that there are advantages of a decrease of a burden on a patient and a very speedy recovery time. It is necessary to suture an incision by using a surgical suture after the endoscopic surgery.

In suturing the incision, a doctor must first knot a distal end of a suture and a doctor must put a lot of time and effort to be skillful at the knotting of a suture. The present invention suggests a structure of a suture and a method of manufacturing the suture, with which a doctor may more easily knot the suture, decrease a surgery time, and safely perform an operation.

Hereinafter, prior arts will be described.

A suture having a distal loop used for an endoscopic surgery was disclosed a long time ago. European Patent No. EP 0559429 A1 discloses a distal loop manufactured using a slip knot, and US Patent No. 5,100,421 suggests an endoscopic surgery using a suture with a distal loop. However, a patent related to a process of manufacturing a suture having such a distal loop is rarely disclosed, and only techniques for a suture having a loop shape for a final shape have been disclosed.

US Patent No. 5,259,846 is characterized in the forming of a loop by connecting a suture to an eye of a needle by simply doubling the suture. However, there is a problem in that the loop is very large to the extent of 760 mm, the suture is thick due to the doubled suture, and the eye of the needle is thick due to an elliptical shape or a shape of an "8" thereof.

The present invention for a patent relating to a method of manufacturing a distal loop using a twist braider, by which the distal loop is manufactured using the twist braider, combining yarn into a single strand and braiding the yarn, and connecting the single yarn to an eye of a needle. Further, the present invention has an advantage in that little damage is created when the suture passes the injured tissue and a thick eye of the needle is not manufactured because the suture is not doubled and thick.

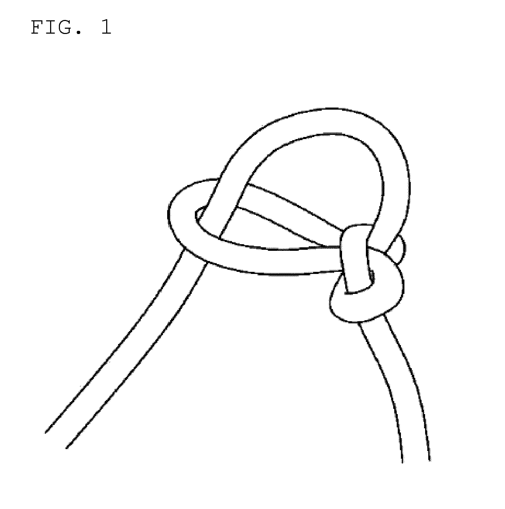

In order to solve the problem of the thick suture, in European Patent No. 0 559 429 A1, a first loop is formed by tying a first knot at a distal end of a suture and a second loop is formed by making the suture pass through the first knot. That is, a slip knot is tied with two different types as illustrated in FIG. 1, and one knot allows the suture to be fixed and the other knot allows the suture to slide through the knot, so that the knot is tightened.

More specifically, European Patent No. 0 559 429 A1 relates to a slip knot. According to a common method of manufacturing a suture, a distal end of single yarn is twisted in a loop shape at a middle region of the single yarn and then the distal end of the suture is tied with a first knot. Then, a second knot is tied such that the knot allows the suture to slide. In this case, when the suture is pulled, the knot allowing the suture to slide is tightened.

However, when the knot is tied at the distal end of the suture, an injury may be disadvantageously caused in sensitive tissue according to penetration of the suture through the tissue. Further, even if the suture is knotted by passing the loop once, the knot becomes thick, so that injured tissue is stimulated to disadvantageously cause the generation of a blister and the continuous application of stimulation during the degradation of the suture.

The present invention does not cause the aforementioned problems at all because the knot is not separately made at the distal loop of the suture. That is, the present invention is an improvement bringing about a smooth connection region of the loop so as to minimize the stimulation applied to the injured tissue during and after surgery.

In order to suture an incision with a surgical suture, once a needle is first passed through a bodily incision, a knot must be created at a distal end of a suture combined with the needle so as to continuously suture the incision. The knot at the end of the suture has a principle similar to that in common sewing in which a knot at a distal end of a suture serves as a stopper after a needle first passes through cloth.

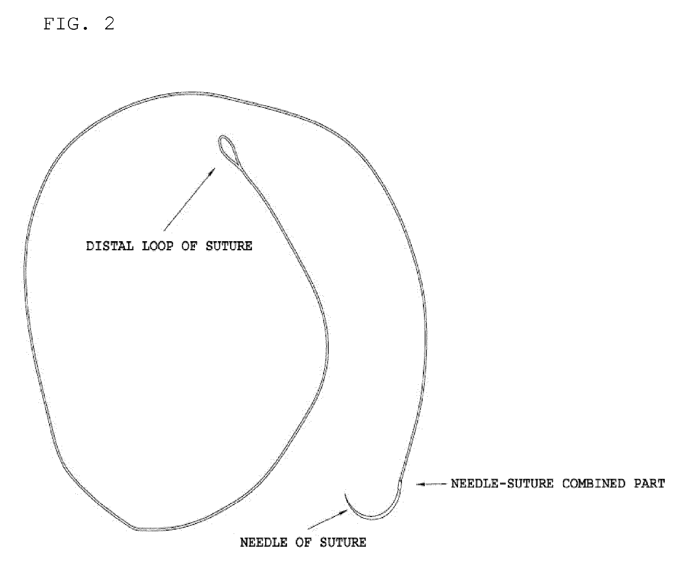

FIG. 2 illustrates a suture (combined with a needle) for suturing an incision according to the present invention.

The present invention relates to a surgical suture, and to a biodegradable surgical suture with a distal loop, which easily creates a knot effect at a bodily region through which a needle passes after the needle passes once through the bodily incision to be sutured without making the knot. More particularly, the present invention relates to the manufacturing of a biodegradable surgical suture with a distal loop, which is characterized in that the distal end is manufactured in a loop shape in manufacturing the surgical suture by using a twist braider.

Specifically, the present invention relates to a method of manufacturing the biodegradable surgical suture with the distal loop by first braiding polyglycolide-co-L-lactide (PGLA) full draw yarn (FDY) and using an upper part of the braided PGLA FDY as a core and braiding it and braiding the PGLA DFY again to manufacture the surgical suture with the distal loop, and then attaching a needle to the surgical suture.

### Detailed description of the invention

### Technical Problem

The present invention relates to a method of manufacturing a surgical suture and an aspect thereof is to suggest a suture structure allowing a doctor to easily perform a suture operation without an essential requisite "skillful knotting technique" in the suture operation. That is, the present invention provides a method of manufacturing a surgical suture having a structure of a loop-shaped distal end, so that it is possible to easily create a knot effect at a bodily region through which a needle passes after the needle passes through a bodily incision to be sutured once without making the knot.

Further, the present invention provides a method of manufacturing a suture having a structure which does not give stimulation and injury to a bodily incision resulting from a knot at a distal end of the suture during and after surgery and does not create concerns on the generation of an infection due to the stimulation and the injury.

### Technical solution

The present invention is conceived to solve the aforementioned problems and achieve the aforementioned aspects.

Especially, the suture having the distal loop structure is introduced into endoscopic surgery, so that the present invention enables a doctor to very easily perform an operation corresponding to making a conventional first knot.

As described above, it is conventionally general that in suturing an incision, a surgical suture is knotted after the surgical suture is passed through injured region once and then the suture is continued. Accordingly, even the one time knotting required the very skillful technique.

However, when the loop, which allows the easy and stable creation of the first knot, is formed at the distal end of the suture according to the present invention, the knot is automatically made when the suture is simply passed through the loop after the suture is passed through injured tissue once, so that it is possible to greatly reduce a surgery time.

That is, a method of manufacturing a surgical suture includes step S10 of preparing multiple bobbins by winding manufactured multifilament or monofilament into the bobbins by using a spinning apparatus; step S20 of putting each bobbin on a carrier of a braider apparatus while yarn wound around the bobbin serves as a sheath 20 and a first core 50; step S30 of twisting sheath-braided yarn 21 or sheath-core braided yarn 22 by braiding the yarn, passing the yarn through a braid collector A, sequentially passing the yarn through take off rollers B and C, a wire eyelet E, and a traverse eyelet F, and winding the yarn with a reel bobbin G; step S40 of cutting a braided yarn wound around the reel bobbin G after the yarn is braided in a predetermined length in step S30; and step S50 of rotating one side of the yarn braided in step S40 by 180° and bending the one side of the yarn (in a downward direction), twisting the yarn in a shape of a loop 25, and passing a straight line-shaped front end of the yarn braided before formation of the loop through a hole of the braid collector A again.

Then, the sheath-core braiding is performed by using the suture, i.e. sheath-braided yarn 21 or sheath-core braided yarn 22, passing the yarn upside down, as the core.

Then, the surgical suture having the distal loop structure is completely manufactured by step S60 of simultaneously braiding the core and the suture in an obliquely downward direction by 45° with respect to a lengthwise direction of the core while surrounding the core.

In the meantime, the method may further include step S70 of completing the suture combined with a needle by inserting the braided suture into an end part of the needle and compressing the needle. In this case, the suture is in a state of being combined with the needle.

The surgical suture is made from the filament manufactured by spinning a homopolymer of a monomer among glycolide, L-lactide, D-lactide, D,L-lactide, p-dioxnaone, epsilon-caprolactone, and trimethylenecarbonate, a copolymer of a combination of glycolide and L-lactide, a copolymer of a combination of L-lactide and epsilon-caprolactone, or a copolymer of a combination of two types among the aforementioned seven monomers, a copolymer of a combination of three types among the aforementioned seven monomers, or a copolymer of a combination of four types among the aforementioned seven monomers, or is made of a material of a copolymer composed with their combination.

The multifilament usable as a material of the surgical suture is made of a homopolymer among polyglycolicacid (PGA), polyglycolic-co-lactide ((PGLA), wherein the lactide contains all of a D-form, an L-form, and a DL-form), polyp-dioxanone (PDO), polyglycolic-co-e-caprolactone (PGCL), polye-caprolactone (PCL), and polytrimethylenecarbonate (PTMC), a copolymer prepared by polymerizing a combination thereof, or one selected from non-biodegradable nylon, polyester, polyethylene, polypropylene, and silk.

The braiding apparatus may include a twist 8 carrier braider, a twist 12 carrier braider, a twist 16 carrier braider, a twist 32 carrier braider, and a twist 64 carrier braider.

### Advantageous Effects

Accordingly, the present invention enables a doctor to very simply and easily make a knot which requires a highly skillful technique in a suture surgery.

To summarize the effects of the present invention, a doctor can very easily and stably tie the surgical suture with the knot which is made after passing injured tissue once in endoscopic surgery, so that it is possible to decrease a surgery time and reduce the pain of a patient, a skillful operation technique is not necessary, and a re-surgery can be prevented according to the stable knot.

That is, since a surface of the distal end of the suture is smooth and there is no unevenly protruding knot, an initial knot in surgery can be very easily made and no stimulation is applied to injured tissue resulting from the forming of the loop at the distal end of the suture, so that it is possible to decrease pain of a patient and prevent infection from being caused.

Further, since only one loop is formed at the distal end of the suture of the present invention, the revolutionary present invention can solve the problems including the time consumption, the requirement of the skillful surgery technique, and a possibility of the generation of tissue injury due to the knot structure of the existing suture at a stroke.

The present invention has remarkable effects below.

That is, a professional doctor very skillful at suturing an injured region was conventionally demanded in endoscopic surgery. However, the loop structure of the present invention enables a doctor to very simply and easily suture the injured region, so that it is possible to solve the problems including many trial and errors required to be a doctor skillful at making a knot, errors in surgery, and much time consumption at the same time.

In a conventional suturing operation using an endoscopy, a doctor should have knotted the suture by using a tool, not by hand, it was very difficult for a general doctor, except for a very skillful doctor, to knot the surgical suture. Especially, a surgeon or a physician spent much time in surgery so as to prevent the knot from being untightened and had large tiredness due to surgery. Further, the suture having the loop structure of the present invention can be stably knotted without untying of the knot, so that a reliability of surgery can be greatly improved.

Further, the surgical suture of the present invention is not doubled and thick, so that an injury generated when the suture is passed through the injured tissue is small and the eye of the needle is manufactured not to have a thick thickness.

### Brief Description of the Drawings

FIG. 1 is a view illustrating a conventional art.
FIG. 2 is a view illustrating a suture combined with a needle according to the present invention.
FIG. 3 is a view of an actual sample of FIG. 2.
FIG. 4 is a picture showing a small bobbin.
FIG. 5 is a diagram schematically illustrating a braiding apparatus.
FIG. 6 is a view illustrating a process of forming a loop.
FIG. 7 is a view schematizing the creation of a knot in suturing a bodily incision by using a surgical suture according to the present invention.

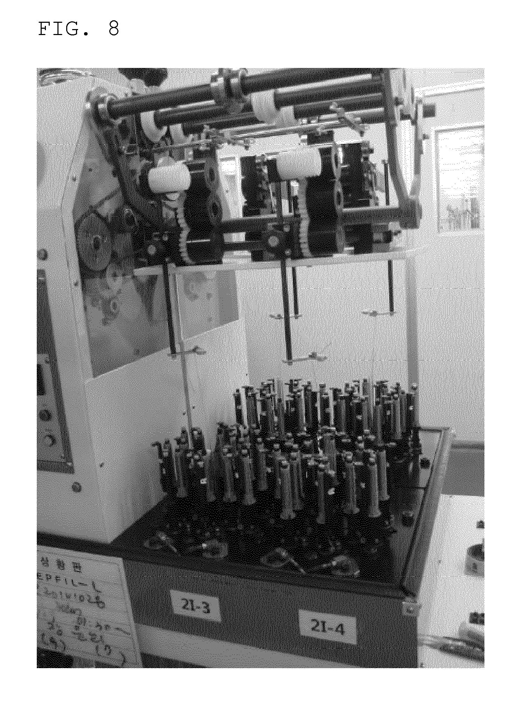

FIG. 8 is a picture showing a whole braiding apparatus.
FIG. 9 is a view illustrating the manufacturing of a suture by braiding a first core and a sheath.

### Detailed description of main reference numbers in drawings

20: sheath
21: sheath braided yarn
25: loop
22: sheath-core braided yarn
40: bodily incision
70: needle

### Best Mode for carrying out the present invention

Hereinafter, exemplary embodiments of the present invention will be specifically described with reference to the accompanying drawings.

However, the technical spirit of the present invention is not limited only to the embodiment of the present invention, and it will appreciate that any invention easily modified by those skilled in the art within the main principle of the present invention belongs to the scope of the right of the present invention.

To first explain terms used in the following description, an abbreviation, "USP", refers to United States Pharmacopeia and an abbreviation, "EP", refers to European Pharmacopeia. An abbreviation, "FDY", refers to full draw yarn and means drawn yarn with multiple strands (approximately 200 to 600 strands) manufactured by melting a polymer chip, inserting the melt polymer chip in a multi spinning apparatus, spinning the polymer chip, and fully drawing the polymer chip, wherein a diameter of one strand is 1.5 µm. An abbreviation, "E.O gas", refers to Ethylene Oxide gas and means sterilized gas.

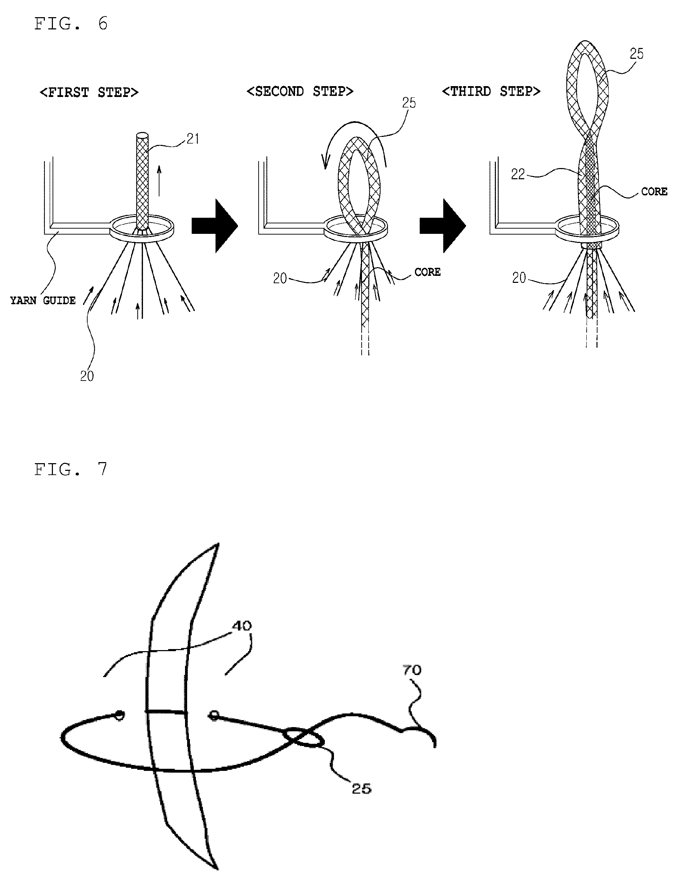

FIG. 7 will be first described in order to help the understanding of the present invention. FIG. 7 illustrates a scene of suturing a bodily incision 40 in a state in which a suture of the present invention is combined with a needle 70. In this case, the suture may be simply tied with a stable knot by making the needle 70 pass through a loop 25.

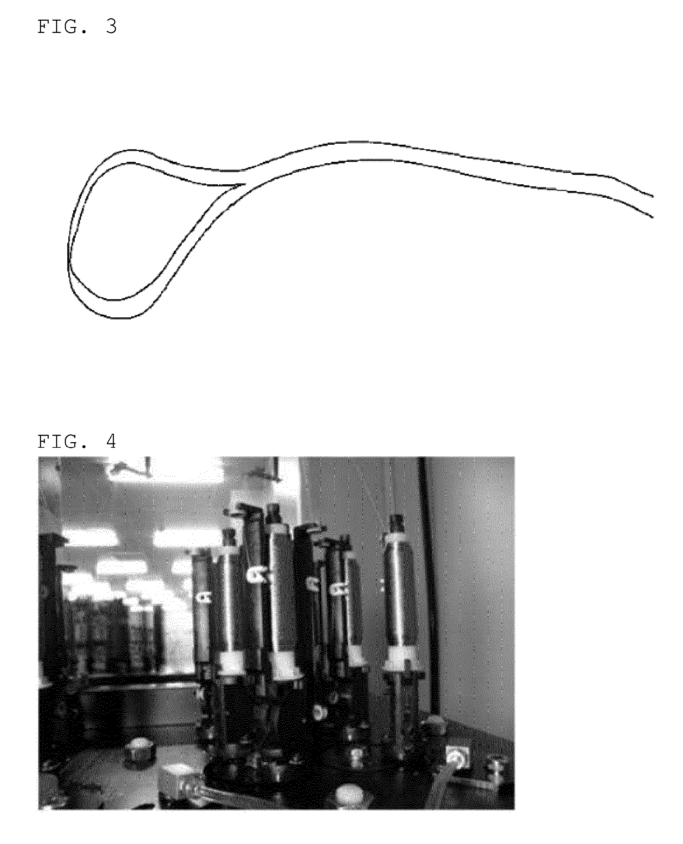

FIG. 2 is a view illustrating the needle-combined surgical suture with a distal end shaped like a loop combined according the present invention, and FIG. 3 is a view of an actually manufactured sample of FIG. 2. The surgical suture illustrated in FIGs. 2 and 3 has the loop-shaped structure at the distal end thereof.

Hereinafter, a method of manufacturing the suture having the loop structure of the present invention will be described.

### Embodiment 1

First, a total of 16 small bobbins was prepared by winding 640 strands of FDY multifilament manufactured by spinning a Poly glycolide-co-L-lactide (PGLA) polymer chip (in a condition of an intrinsic viscosity of 1.6 dl/g, a temperature of 30°C, 0.1% of Hexafluoroisopropanol (HFIP) solution) within a multi spinning apparatus to each of the small bobbins by approximately 1 km.

Next, each small bobbin wound with the filament is put on a carrier of a twist braider while the filament wound around the small bobbin serves as the sheath. FIG. 4 is a picture showing the small bobbins put on the twist braiders through the aforementioned processes.

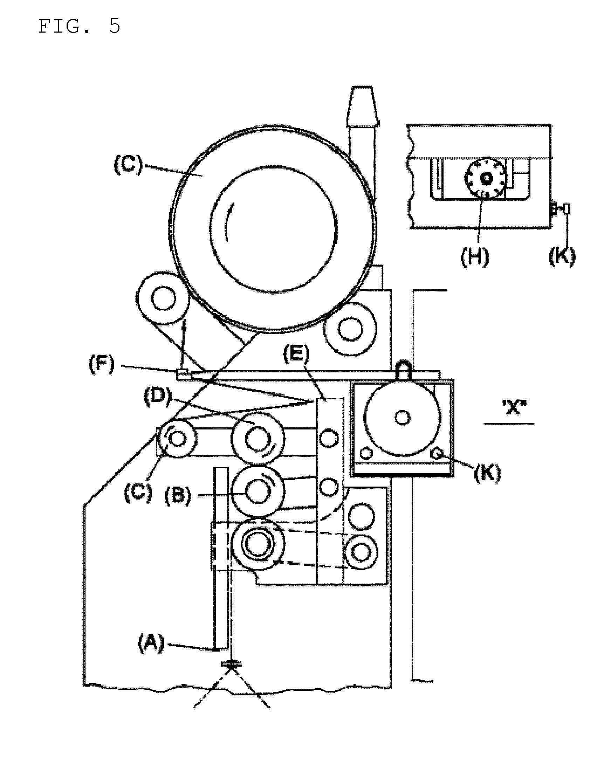

A process after the completion of the aforementioned preparation will be described (see FIGs. 5 and 6). FIG. 5 illustrates a device for braiding the yarn wound around the small bobbin, and the device is referred to as the twist braider.

That is, after the FDY multifilament wound around the small bobbin is passed through a braid collector A, the FDY multifilament is sequentially passed through take off rollers B and C, a wire eyelet E, and a traverse eyelet F and then is wound with a reel bobbin G.

Subsequently, the yarn is braided in a predetermined length (preferably, 85 cm), followed by cutting the braided yarn wound around the reel bobbin G.

### First step

In the first step, a sheath braiding for simply twisting the yarn wound around the small bobbins is performed. The sheath braiding refers to an operation of twisting thready yarn wound around the small bobbin.

### Second step

In the second step, one side of a center of the sheath-braided yarn 21 or sheath-core braided yarn 22 prepared in the first step is rotated by 180° and bent (u-turned) (in a downward direction), the one side is then twisted in a shape of the loop 25, followed by passing a straight line-shaped front end (core) braided before the formation of the loop through a hole of the braid collector A again. FIG. 6 illustrates the process of forming the loop for more easy understanding.

### Third step

In the third step, the sheath-core braiding is performed for the core, and in this case the sheath-core braiding is performed based on the core in a downward direction. In this case, the use of the sheath-braided yarn 21 or the sheath-core braided yarn 22 as the core means that the sheath-braided yarn 21 or the sheath-core braided yarn 22 is used as a center wick such that the yarn is wound around the sheath-braided yarn 21 or sheath-core braided yarn 22.

Reference number 22 is a sheath-core braid, and refers to a combination of the sheath-braided yarn and the core that is the already braided yarn at a center of the sheath-core braid.

In the process of the manufacturing, it is preferable to braid the sheath and the core of approximately 800 mm, but the length of the suture is not limited to 800 mm and may be variously changed.

Next, the surgical suture with the distal end shaped like the loop is finally completed by washing the manufactured yarn with methanol and attaching a needle to the distal end. In this case, the needle is provided with a seating recess such that the yarn is seated. Accordingly, the surgical suture is finally completed by inserting the manufactured yarn in the seating recess and compressing the end of the needle.

Further, in the process, an external diameter of the distal loop of the surgical suture is preferably 7 mm and a total length of the suture is 480 mm, but they are not limited thereto, and the loop may be formed in a size larger or smaller than 7 mm, and a diameter of the loop may range from 1 mm to 100 mm depending on the usage.

In the meantime, it is characterized in that the surgical suture is made from the filament manufactured by spinning a homopolymer of a monomer among glycolide, L-lactide, D-lactide, D,L-lactide, p-dioxnaone, epsilon-caprolactone, and trimethylenecarbonate, a copolymer of a combination of glycolide and L-lactide, a copolymer of a combination of L-lactide and epsilon-caprolactone, a copolymer of a combination of two types among the aforementioned seven monomers, a copolymer of a combination of three types among the aforementioned seven monomers, or a copolymer of a combination of four types among the aforementioned seven monomers, or is made of a material of a copolymer composed with their combination.

### Embodiment 2

In embodiment 2, a material of the yarn of embodiment 1 is different. That is, the suture is made from the FDY multifilament manufactured by spinning a polymer chip made of a raw material of Polyglycolide (PGA) (in a condition of an intrinsic viscosity of 1.5 dl/g, a temperature of 30°C, 0.1% of Hexafluoroisopropanol (HFIP) solution) within a multi spinning apparatus, followed by the process of forming the loop, to obtain the completed surgical suture.

In the meantime, an apparatus for manufacturing the distal loop of the surgical suture of the present invention is not limited to the twist braider. That is, the apparatus may include a twist 2 to 252 carrier braider including a twist 8 carrier braider, a twist 12 carrier braider, a twist 16 carrier braider, a twist 32 carrier braider, and a twist 64 carrier braider.

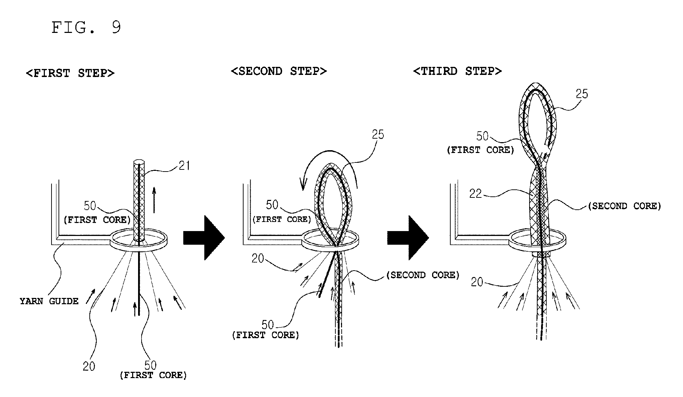

### Embodiment 3 (see FIG. 9)

Embodiment 3 is different from embodiment 1 in that "a sheath-first core braiding" is performed instead of "the sheath braiding" performed in the first step of embodiment 1.

FIG. 9 illustrates the steps of manufacturing of the suture by braiding a first core and a sheath, and the second step and the third steps of FIG. 9 are performed in the same manner as those of embodiment 1.

The term "the first core" means that several strands of sheath are braided based on the first core. A strand thickly drawn in the first step of FIG. 9 corresponds to the first core 50.

Further, the term "the core" used in embodiment 1 has the same meaning as that of "a second core" in present embodiment 3.

Referring to FIG. 9, after performing the sheath-first core braiding in the first step, the sheath-first core braided yarn is twisted in a loop shape in the second step, and the braiding (sheath-second core braiding) is performed while using the state (the front end of the loop) of the second step as the second core in the third step.

The processing method of the sheath-second core braiding is substantially the same as that of the aforementioned sheath-core braiding.

The first core may include 50 strands of PGLA FDY filament, and also include other biodegradable multifilament. Further, the sheath may include monofilament or multifilament.

### Embodiment 4

Embodiment 4 is the same as embodiment 3 except for the use of monofilament as the first core, contrary to the multifilament as the first core in embodiment 3.

It is preferable to use PDO USP 6-0 monofilament as the first core in embodiment 4, but other biodegradable monofilament may also be used.

### Embodiment 5

Embodiment 5 is characterized in that braided mixed multifilament and monofilament are used as "the first core". The remaining manufacturing processes are the same as those of embodiment 3.

That is, in the aforementioned embodiments, the first core 50 may be made from any one between the multifilament and the monofilament in step S20, and the sheath 20 may be made from any one between the multifilament and the monofilament.

Further, in step S20, the first core 50 is manufactured by mixing the multifilament and the monofilament, and the sheath 20 may be manufactured by any one between the monofilament and the multifilament or by mixing the multifilament and the monofilament.

In the meantime, a property of the suture according to the present invention was measured as follows.

### 1) Diameter measurement method

A diameter of the suture was measured in a scheme of USP 921, and measured by applying tension to the suture of the multifilament with a measurement device, a Mitutoyo Dial Gage. Further, the suture was measured once and then measured again by rotating the suture by 90°, and an average of 10 measurement values was calculated.

### 2) EP knot measurement method

The surgical suture was knotted once by using a tension strength measurement device made by Instron Company and an average of measurement values of five strands in the EP simple knot method was obtained.

### 3) Knot strength maintenance rate measurement method

Strength of an initial EP knot of the manufactured suture was measured. Then, 1M phosphate buffered saline having 7.4 pH was prepared. The manufactured suture in a length of approximately 2 m was put into a tube for a test and the tube was filled with the 1M phosphate buffered saline such that the suture was submerged. The prepared tube for test was inserted into a shaking bath adjusted to have 37 °C and taken out from the tube after 14 days, followed by measuring the EP knot strength, to calculate a knot strength maintenance rate.

The values of the properties measured through the aforementioned methods are as follows.

**Table 1**

| Embodiment | Diameter (mm) | Diameter of distal loop (mm) | External diameter of distal loop (mm) | EP knot strength (kgf/cm2 ) | Strength maintenance rate for two weeks (%) |
|---|---|---|---|---|---|
| Embodiment 1 | 0.537 | 0.425 | 8 | 7.53 | 87 |
| Embodiment 2 | 0.535 | 0.423 | 8 | 10.45 | 85 |
| Embodiment 3 | 0.540 | 0.425 | 8 | 10.60 | 87 |
| Embodiment 4 | 0.647 | 0.535 | 10 | 10.95 | 88 |

As described above, in the present invention, the method of manufacturing the biodegradable surgical suture with the very smooth distal loop, which is used for endoscopic surgery, by using the twist braider was developed.

In suturing an incision in endoscopic surgery, in a case where the loop, which causes the surgical suture to be easily knotted after the surgical suture passes the incision once, is formed at the distal end of the suture in advance, when the surgical suture is passed through injured tissue once and then simply passes through the loop, the surgical suture is automatically knotted, so that a surgery time may be decreased.

Further, since the knotting requiring the highly skillful technique is accomplished once, errors in the knotting in endoscopic surgery may be decreased.

As a result, when the biodegradable suture having the distal loop structure according to the present invention is applied to endoscopic surgery, it is possible to greatly decrease time and expenses and the skillful technique related to the knotting of the suture is not necessary.

### Industrial applicability

The method of manufacturing the surgical suture of the present invention is applied to a method of manufacturing a suture for various surgical operations, such as plastic surgery, fixing of skin, suturing of soft tissue, and the fixing of an implant to tissue.

## Claims

1. A biodegradable suture with one end having a loop structure without a knot and another end combined with a needle, wherein a part other than the one end is twist braided to be formed in a single strand.

2. A method of manufacturing a surgical suture, comprising:
step S10 of preparing multiple bobbins by winding manufactured multifilament or monofilament into the bobbins by using a spinning apparatus;
step S20 of putting each bobbin on a carrier of a braider apparatus while yarn wound around the bobbin serves as a sheath 20 and a first core 50;
step S30 of twisting sheath-braided yarn 21 or sheath-core braided yarn 22 by braiding the yarn, passing the yarn through a braid collector A, sequentially passing the yarn through take off rollers B and C, a wire eyelet E, and a traverse eyelet F, and winding the yarn with a reel bobbin G;
step S40 of cutting a braided yarn wound around the reel bobbin G after the yarn is braided in a predetermined length in step S30;
step S50 of rotating one side of the yarn braided in step S40 by 180° and bending the one side of the yarn (in a downward direction), twisting the yarn in a shape of a loop 25, and passing a straight line-shaped front end of the yarn braided before formation of the loop through a hole of the braid collector A again; and
step S60 of performing a sheath-second core braiding in a downward direction for a part (second core) of the yarn passed through the hole of the braid collector in step S50.

3. A method of manufacturing a surgical suture, comprising:
step S100 of preparing multiple bobbins by winding manufactured multifilament or monofilament into the bobbins by using a spinning apparatus;
step S200 of putting each bobbin on a carrier of a braider apparatus while yarn wound around the bobbin serves as a sheath 20 and a first core 50;
step S300 of twisting sheath-braided yarn 21 by braiding the yarn, passing the yarn through a braid collector A, sequentially passing the yarn through take off rollers B and C, a wire eyelet E, and a traverse eyelet F, and winding the yarn with a reel bobbin G;
step S400 of cutting the braided yarn wound around the reel bobbin G after the yarn is braided in a predetermined length in step S300;
step S500 of rotating one side of the yarn braided in step S400 by 180° and bending the one side of the braided yarn (in a downward direction), twisting the yarn in a shape of a loop 25, and passing a straight line-shaped front end of the yarn braided before formation of the loop through a hole of the braid collector A again; and
step S600 of performing a sheath-core braiding in a downward direction for a part (second core) of the yarn passed through the hole of the braid collector in step S500.

4. The method as claimed in claim 2 or 3, wherein the surgical suture is made from the filament manufactured by spinning a homopolymer of a monomer among glycolide, L-lactide, D-lactide, D,L-lactide, p-dioxnaone, epsilon-caprolactone, and trimethylenecarbonate, a copolymer of a combination of glycolide and L-lactide, a copolymer of a combination of L-lactide and epsilon-caprolactone, or a copolymer of a combination of two types among the aforementioned seven monomers, a copolymer of a combination of three types among the aforementioned seven monomers, or a copolymer of a combination of four types among the aforementioned seven monomers, or is made of a material of a copolymer composed with their combination.

5. The method as claimed in claim 2 or 3, wherein the multifilament as a material of the surgical suture is made of a homopolymer among polyglycolicacid (PGA), polyglycolic-co-lactide ((PGLA), wherein the lactide contains all of a D-form, an L-form, and a DL-form), polyp-dioxanone (PDO), polyglycolic-co-e-caprolactone (PGCL), polye-caprolactone (PCL), and polytrimethylenecarbonate (PTMC), a copolymer prepared by polymerizing a combination thereof, or one selected from non-biodegradable nylon, polyester, polyethylene, polypropylene, and silk.

6. The method as claimed in claim 2 or 3, wherein the braider apparatus uses a twist 2 to 252 carrier braider.

7. The method as claimed in claim 2 or 3, further comprising step S70 of completing the surgical suture combined with a needle by inserting the yarn braided in step S60 or S600 into an end part of the needle and compressing the needle.

8. The method as claimed in claim 2, wherein in step S20, the first core 50 is manufactured using one of the multifilament and the monofilament and the sheath 20 is manufactured using one of the multifilament and the monofilament.

9. The method as claimed in claim 2, wherein in step S20, the first core 50 is manufactured mixing the multifilament and the monofilament, and the sheath 20 is manufactured using one of the multifilament and the monofilament or by mixing the multifilament and the monofilament.

10. The method as claimed in claim 3, wherein the sheath 20 is manufactured using one of the multifilament and the monofilament or by mixing the multifilament and the monofilament.
